# EUROPEAN PATENT APPLICATION

(11) **EP 4 459 286 A1**
(43) Date of publication of application: **06.11.2024**
(21) Application number: 22915160.0
(22) Date of filing: 30.12.2022
(51) Int. Cl.: G01N 33/577, G01N 33/543, G01N 33/53, G01N 21/31

(54) **KIT FOR QUANTITATIVELY DETERMINING TACROLIMUS IN WHOLE BLOOD BY LOW-RESISTANCE LATEX-ENHANCED IMMUNE COMPETITION METHOD, AND USE THEREOF**

(30) Priority: 30.12.2021 CN 202111660017
(71) Applicant: SHANGHAI YUNZE BIOTECHNOLOGY CO., LTD, Shanghai 201112 (CN); SHANGHAI GENEXT MEDICAL TECHNOLOGY CO., LTD, Shanghai 201114 (CN)
(72) Inventor: LIN, Weijing, Shanghai 201112 (CN); SONG, Xiaochan, Shanghai 201112 (CN); QIN, Linjuan, Shanghai 201112 (CN)
(74) Representative: Greaves Brewster LLP
(86) International application number: PCT/CN2022/143841
(87) International publication number: WO 2023/125911

(57) **Abstract**

Disclosed are a kit for quantitatively determining tacrolimus in whole blood by a low-resistance latex-enhanced immune competition method, and a use thereof. The kit comprises a sensitizing latex reagent, the sensitizing latex reagent comprising a latex microsphere-NTA-Ni-Fc receptor-anti-tacrolimus monoclonal antibody. Further disclosed is a method for quantitatively determining the tacrolimus concentration in a sample. The kit for determining the immunosuppressant, tacrolimus, in whole blood by using a latex-enhanced immune competition method provided by the present invention has advantages such as a simple and convenient measuring method, low antibody steric hindrance, and high sensitivity.

## Description

The present application claims the priority of Chinese patent application 2021116600176, filed on December 30, 2021. The contents of the above patent application is incorporated herein by reference in its entirety.

### TECHNICAL FIELD

The present invention relates to the field of biochemical detection, specifically relates to a kit for quantitatively determining tacrolimus in whole blood by low-resistance latex-enhanced immune competition method, and use thereof.

### BACKGROUND

Tacrolimus (FK506, also known as: Prograf, tacarolimus) is a macrolide antibiotic extracted from soil fungi, with strong immunosuppressive properties. Its drug strength is 10-100 times than that of cyclosporine A, and its effect of preventing rejection in various organ transplants is superior to that of cyclosporine.

In 1984, *Streptomyces tsukuba* was isolated by Fujisawa of Japan, in the Tsukuba area of Osaka, Japan, and the component tacrolimus was obtained by fermentation, purification, and isolation. Prograf was first on trial clinically in 1989 at the University of Pittsburgh Starzl Transplantation Institute in the United States. In 1993, it was launched in Japan as Prograf. It was officially used in various countries after being approved by the FDA in 1995. At present, Prograf has been widely used in the transplantation of solid organs such as liver, pancreas, kidney, heart and lung. According to a report from the University of Pittsburgh of the United States, among rescue therapies that are ineffective with cyclosporine, the success rate of using FK506 in liver transplantation patients was 87%, while that in kidney transplantation patients was 74%. Since its launch in China in 1999, the cumulative number of transplantation patients who have used this product has exceeded 5000 so far, and almost every transplantation center and institute uses Prograf.

The efficacy and adverse effects of tacrolimus are closely related to its blood drug concentration. The blood drug concentration may increasethe incidence of adverse effects when it is too high, and may increase the risk of rejection when it is too low. Therefore, the blood drug concentration of tacrolimus should be controlled in the range of optimal efficacy and the lowest adverse effects. Individualized drug therapy is guided by therapeutic drug monitoring (TDM), where the dosage should be determined individuallyand the optimal dosage is determined based on the specific condition of different patientsstriving for the best effect and minimizing adverse effects.

At present, the main methods for detecting the blood drug concentration of FK506 domestically include high-performance liquid chromatography-mass spectrometry (HPLC-MS), enzyme-magnification immunoassay (EMIA), chemiluminescence microparticle immunoassay (CMIA), electrochemiluminescence immunoassay (ECLIA), and so on. One of the methods, HPLC-MS, is accurate and sensitive for determining blood drug concentrations, but it is complicated to operate, time-consuming and costly to detect, requiring expensive equipment, and mainly used in scientific research fields or as a reference method. The domestic market is basically shared by Siemens EMIA, Abbott CMIA, and Roche ECLIA, all of which offer methodologies with highly automatic and accurate results for detection. However, the reagents are expensive and the use of specialized instruments is required.

### NTA-Ni-His tag

In 1987, Hochuli et al. invented the improved metal chelate ligand nitrilotriacetic acid (NTA). With the rapid development of modern molecular biology and recombinant protein technology, NTAligands have been significantly used in the purification of his-tagged proteins.

It is well known that the widely used metal ion is Ni²⁺, which has a total of six coordination sites around it for ligand binding. IDA is a tridentate ligand, i.e., it can bind to three coordination sites around Ni²⁺, while two of the remaining three coordination sites are used to bind to histidine residues in histidine-tagged proteins and one is used to coordination binding with H₂O. Therefore, IDA has relatively weak binding ability to Ni²⁺, resulting in the easy detachment of Ni²⁺ bound to a medium, which leads to unstable performance of the medium. NTA is a tetridentate ligand, i.e., it can bind to four coordination sites around Ni²⁺, while the remaining two coordination sites are used to bind to histidine residues in histidine-tagged proteins.

### Fc receptor protein

Fc receptors refers to a class of receptors on the cell membrane surface that can bind to IgFc fragments. In immunology, cells with Fc receptors generally include B cells, killer cells, and macrophages. Antibodies are special proteins produced by immune cells that can bind to antigens such as microorganisms, toxins, or allergens. When the Fc receptors on immune cells bind to antibodies and their attached antigens, phagocytosis is triggered, which consumes the antigen coated by the antibodies.

Based on different types of antibodies, there exist different specific antigens. This also means that there are different Fc receptors, each of which can bind to the Fc region of an antibody. Antibodies are also known as immunoglobulins, abbreviated as Ig, and the most common type found in the blood is IgG. Fc receptors are mainly divided into three categories: Fc-γ receptors, Fc-α receptors, and Fc-ε receptors. Each type of Fc receptor includes several subtypes based on genetic homology, such as Fc-γ receptors can bind to the Fc fragment of IgG, Fc-α receptors can bind to the Fc fragment of IgA, and Fc-ε receptors can bind to the Fc fragment of IgE. In recent years, it has been found in researches that a murine Fc receptor (Fcα/µR, also known as FcAMR, CD351) can bind to both IgM and IgA.

Latex-enhanced turbidimetric immunoassay method has advantages of simple operation, rapidity, high sensitivity, applicability to automatic biochemical analyzers, and ease of domestication. However, latex-enhanced turbidimetric immunoassay method is mostly used for the determination of large molecular proteins, and has the disadvantage of insufficient sensitivity compared to the methodologies of CMIA and ECLIA. Therefore, the development of a novel latex-enhanced immune method with higher sensitivity for detecting the small molecule drug tacrolimus will provide strong support for the clinical use of Tacrolimus as an immunosuppressant.

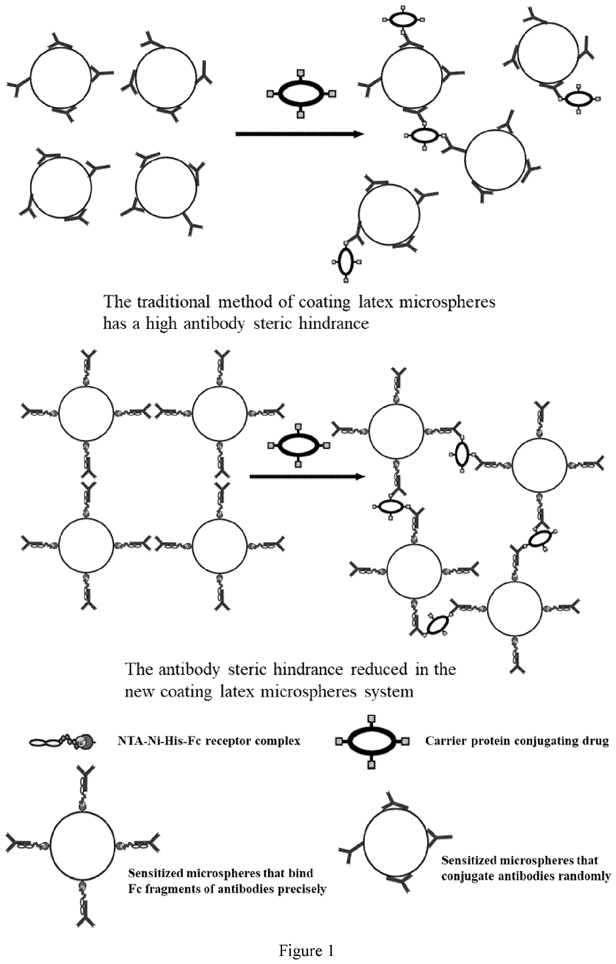

Latex-enhanced immune competitive method is currently a commonly used method for detecting the concentration of small drug molecules in samples. The principle of this method is to cross-link monoclonal antibodies on the surface of polymer latex microspheres. The drug molecule (the hapten in the samples) and the drug-protein complex (artificially constructed antigen) compete simultaneously for the binding to the antibody sites on the surface of the latex microspheres. The turbidity of the reaction system is inversely related to the concentration of the small drug molecules in the samples. By measuring the absorbance of the reaction solution, the concentration of the small drug molecules in the samples can be calculated. However, the traditional method of coating latex microspheres has a high antibody steric hindrance, as shown in Figure 1.

### CONTENT OF THE INVENTION

The technical problem that the present invention aims to address is the complexity, low sensitivity, and high antibody steric hindrance in the existing methods for detecting small molecule drug tacrolimus. Provided is a kit using the latex-enhanced immune competitive method for quantitatively determining the immunosuppressive drug tacrolimus in whole blood with low resistance and high sensitivity, and use thereof. The specific principle is as follows:

Before determining the concentration of tacrolimus, the whole blood sample is pretreated to extract tacrolimus that is bound to intracellular proteins in the whole blood. During the detection process, the extracted tacrolimus from the sample competes with the tacrolimus drug-protein complex in the drug-protein complex reagent (Reagent R1) to bind to anti-tacrolimus antibodies on the surface of latex microspheres in the sensitizing latex reagent (Reagent R2). The competitive binding of the small molecule drug of tacrolimus in the sample for the antibody sites inhibits the aggregation reaction. Therefore, when the concentration of tacrolimus in the sample is low, a stronger aggregation reaction occurs; when the concentration is high, a weaker aggregation reaction occurs. The turbidity of the reaction system is inversely related to the concentration of tacrolimus in the sample, and the concentration of tacrolimus in the whole blood sample is calculated by measuring the absorbance near 546 nm.

Another innovative aspect of the present invention is nanolatex microspheres that are conjugated with anti-tacrolimus monoclonal antibodies used in the present invention. These microspheres can direct-bind to the Fc fragment of antibodies, effectively reduce the antibody steric hindrance and fully expose the active sites of antibodies to enhance sensitivity, the schematic diagram of which is as shown in Figure 1.

The anti-tacrolimus monoclonal antibodies used in the present invention can be prepared using the method described in Example 2 and the most effective antibodies can be selected for use in the present invention. However, the anti-tacrolimus monoclonal antibodies available for use in the present invention are not limited to these. Any anti-tacrolimus monoclonal antibodies that can be purchased through commercial channels and the type of which corresponds to the specific Fc receptor protein can achieve technical effects equivalent to those of the present invention.

To address the aforementioned technical problems, the first technical solution provided by the present invention is: a kit for quantitatively determining tacrolimus, comprising a sensitizing latex reagent, wherein the sensitizing latex reagent comprising a latex microsphere-NTA-Ni-Fc receptor-anti-tacrolimus monoclonal antibody.

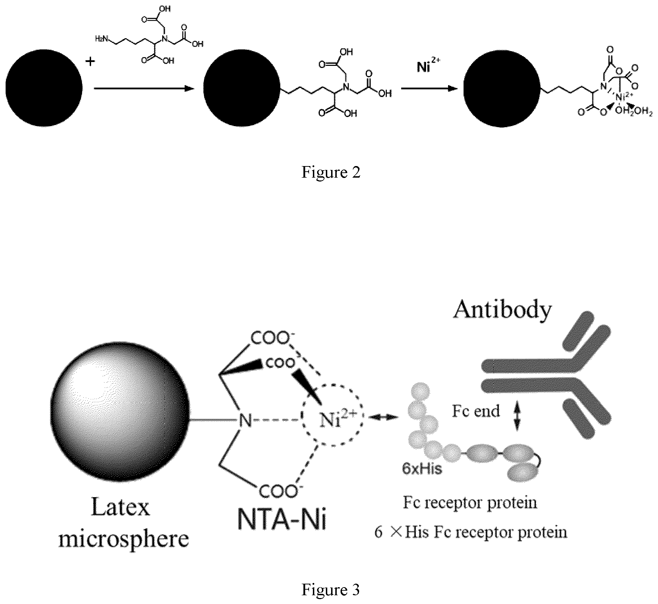

The kit of the first technical solution, wherein the preparation method of the latex microsphere-NTA-Ni-Fc receptor-anti-tacrolimus monoclonal antibody comprises following steps, with a schematic representation of the reaction as shown in Figure 2 and Figure 3:
i) binding a latex microsphere covalently with N-(5-amino-1-carboxypentyl) iminodiacetic acid (with an NTA structure at the end) to produce latex microsphere-NTA.
ii) binding the latex microsphere-NTA with a Ni ion to produce latex microsphere-NTA-Ni.
iii) binding the latex microsphere-NTA-Ni with a His-tagged Fc receptor (also called FcBP, Fc-binding protein, which has a variety of structures, and there are many kinds of them commercially available currently, which are easy to obtain, and different kinds correspond to different antibody types, e.g., FcγR1 and FcAMR) to produce latex microsphere-NTA-Ni-Fc receptor.
iv) binding the latex microsphere-NTA-Ni-Fc receptor with the Fc end of an anti-tacrolimus monoclonal antibody to produce the latex microsphere-NTA-Ni-Fc receptor-anti-tacrolimus monoclonal antibody.

According to conventional practice in the art, there are two types of surface groups could be selected for the latex microsphere used in the present invention: carboxyl and amino. As long as an appropriate ratio and coupling agent are chosen, the same effect can theoretically be achieved, i.e., both types of microspheres can be used in the present invention.

The kit of the first technical solution, when the latex microsphere is a carboxyl latex microsphere, NHS (N-hydroxysuccinimide) and EDC (1-ethyl-(3-dimethylaminopropyl) carbodiimide) are used as coupling agents, and the preparation of the latex microsphere-NTA of i) comprises following steps:
(1) dispersing the carboxyl latex microsphere with a particle size of 50 nm to 350 nm in MES (morpholine ethanesulfonic acid) with a pH of 5.8 to 6.5, making the microsphere mass volume percentage at 1%, then adding N-hydroxysuccinimide and 1-ethyl-(3-dimethylaminopropyl) carbodiimide at a coating concentration of 0.1 to 3 mg/10 mg of microsphere, and reacting at room temperature for 0.5 to 3 hours; the pH is preferably 6.1; the time of the recation at room temperature is preferably 1 hour, and the coating concentration of N-hydroxysuccinimide and 1-ethyl-(3-dimethylaminopropyl)carbodiimide is preferbaly 1-1.5 mg/10 mg of microsphere.
(2) Centrifuging and discardingthe supernatant, and resuspending with ultrasound in MES with pH of 5.8 to 6.5; the pH is preferably 6.1.
(3) Adding 0.5-3 mg/mL, such as 1.2 mg/mL, of N-(5-amino-1-carboxypentyl) iminodiacetic acid, stirring and reacting for 1-3 h.
(4) Adding excessive 30-50 mmol of primary amine (e.g., hydroxylamine, ethanolamine, glycine) and block-reacting for 1- 3h.
(5) Centrifuging and discarding the supernatant, and resuspending with ultrasoundin PBS at a pH of 6.8 to 8.5, to produce the latex microsphere-NTA; the PBS is preferably at pH 7.4, 100 mM.

The kit of the first technical solution, wherein the preparation of the latex microsphere-NTA-Ni of ii) comprises following steps:
(1) adding excessive Ni(CH₃COO)₂, stirring and reacting for 0.5-3 h; preferably 2 h.
(2) Centrifuging and discarding the supernatant, resuspending withultrasound in PBS at a pH of 6.8 to 8.5, storing at 2-8°C for later use; the PBS is preferably at pH 7.4, 100 mM. There is no need to add preservativesfor short-term preservation, but preservatives need to be added for long-term preservation. The preservatives can be sodium azide or thimerosal.

The kit of the first technical solution, wherein the preparation of the latex microsphere-NTA-Ni-Fc receptor of iii) comprises following steps:
(1) adding the His-tagged Fc receptor to the latex microsphere-NTA-Ni and reacting for 0.5-3 h; preferably 2 h.
(2) Centrifuging and discarding the supernatant, suspending with ultrasound in PBS, and disolving in PBS after washing twice repeatedly.

According to common practice in the art, the His-tagged Fc receptor can be a 6×His-Fc receptor.

The kit of the first technical solution, wherein the preparation of the latex microsphere-NTA-Ni-Fc receptor-anti-tacrolimus monoclonal antibody of iv) comprises following steps:
(1) adding the anti-tacrolimus monoclonal antibody to the latex microsphere-NTA-Ni-Fc receptor, making the antibody coating amount of the latex microsphere at 0.05 to 10 mg of anti-tacrolimus monoclonal antibody /10 mg of latex microsphere, and reacting for 1 hour; the antibody coating amount of the latex microsphere is preferably 0.1 to 0.4 mg of anti-tacrolimus monoclonal antibody /10 mg of latex microsphere.
(2) Centrifuging and discarding the supernatant, suspending with ultrasound in PBS, washing twice repeatedly and dissolving in a microsphere storage solution.

The kit of the first technical solution, wherein the microsphere storage solution comprises a buffer with a concentration of 50-500 mM and a pH of 6.5-7.8, an ionic stabilizer with a mass percentage of 0.5-5%, a microsphere stabilizer with a mass percentage of 0.05-0.5%, and a preservative.

According to common practice in the art, the buffer can be Tris, HEPES, or PBS; the ionic stabilizer can be NaCl, KCl, or MgCh; and the preservative can be sodium azide, thimerosal, or ProClin300.

The buffer is PBS with a concentration of 100 mM and a pH of preferably 7.4; the ionic stabilizer is NaCl with the mass percentage preferably 2%; the microsphere stabilizer is glycine with the mass percentage preferably 0.1% to 0.2%; the preservative is preferably sodium azide.

The speed and time of centrifugation described herein may be conventional in the art, e.g., centrifuging at 12,000 rpm for 30 min.

The kit of the first technical solution, wherein the kit further comprises a drug-protein complex reagent, which comprises a tacrolimus drug-protein complex, a stabilizer, a coagulant promoter, a surfactant, a preservative and a buffer.

The concentration of the tacrolimus drug-protein complex is 0.1 µg/mL-3 µg/mL; preferably 1 µg/mL.

The buffer can be conventional in the art, such as Tris, HEPES, PBS, preferably HEPES; the concentration is 50-500 mM, the pH is 6.5-7.8, preferably 50 mM, pH 6.8.

The stabilizer can be conventional in the art, such as one or a combination of more of glycerol, trehalose, mannitol, and dextran, with a mass percentage of 1% to 10%.

The surfactant can be conventional in the art, such as one or a combination of two of Tx100, Tween20, Tween80, Span20, Span60, and Span80, with a mass percentage of 0.01% to 0.5%.

The coagulant promoter can be one or a combination of more of PEG6000, PEG8000, PEG20000, and PEG35000.

The preservative can be sodium azide, thimerosal, or ProClin300.

The kit of the first technical solution, wherein the kit further comprises a calibrator, which comprises a preservative, a tacrolimus pure product, and a bovine whole blood matrix, and the concentration of tacrolimus in the calibrator is selected from 5 to 7 points between 0-50 ng/mL.

In a preferred embodiment of the present invention, the concentrations of tacrolimus in the calibrator are 0 ng/mL, 3 ng/mL, 6 ng/mL, 10 ng/mL, 20 ng/mL, and 30 ng/mL, respectively, 6 points in total.

The preservative can be one or a combination of more of sodium azide, thimerosal, ProClin300, and gentamicin.

The kit of the first technical solution, wherein the kit further comprises a sample pre-treatment agent, which comprises a protein denaturant, a protein hydrolase, a surfactant and a buffer.

The buffer is Tris-HCl buffer with a pH of 6.5-8.5.

The protein denaturant is uric acid of 3 mol/L-8 mol/L, and/or guanidine hydrochloride of 2 mol/L-6 mol/L.

The protein hydrolase is subtilisin of 2.5 U/ml-10 U/ml.

The surfactant can be one or a combination of more of SDS, Tx100, and Tween20.

To address the aforementioned technical problems, the second technical solution provided by the present invention is: a method for quantitatively determining the concentration of tacrolimus in a sample, wherein the method comprises using the kit of the first technical solution, and the method comprises following steps:
(1) mixing and incubating the sample or the calibrator with the sample pre-treatment agent at a volume of 1:1, and centrifuging to obtain a supernatant (principle: tacrolimus is released for a state of free by denaturation of the proteins bound with small molecule tacrolimus with a denaturant in the whole blood sample or the calibrator); the water bath incubation is preferably incubation in water bath at 42°C for 10 minutes.
(2) mixing the supernatant in (1) with the drug-protein complex reagent and incubating to obtain a mixture; the incubation is preferably performed at 37°C for 3 to 6 minutes.
(3) adding the sensitizing latex reagent to the mixture in (2), reading the absorbance as A1, incubating, and reading the absorbance as A2; the incubation is preferably performed at 37°C for 3 to 6 minutes.
(4) calculating the absorbance increment ΔA of the sample or the calibrator, ΔA = A2-A1.
(5) establishing a calibration curve with the absorbance increment ΔA of the calibrator against the concentration of tacrolimus contained, and achieving quantitative detection of the concentration of tacrolimus in the sample by comparing the absorbance increment ΔA of the sample.

The characteristic of this detection method is that the small molecule of the drug to be determined in the sample competes with the drug-protein complex to bind to the antibody sites on the surface of the sensitized latex microspheres. The more small molecules of the drug to be determined in the sample, the more distinct inhibitory effect on the agglutination reaction; thereby realizing the determination of the drug concentration.

On the basis of complying with common knowledge in the art, the above-mentioned preferred conditions can be arbitrarily combined to obtain the preferred examples of the present invention.

Reagents and raw materials used in the present invention are commercially available.

The positive effect of the present invention is: the kit provided by the present invention for determining the immunosuppressant, tacrolimus, in whole blood by using latex-enhanced immune competitive method has advantages such as a simple and convenient detection method, low antibody steric hindrance, and high sensitivity.

### BRIEF DESCRIPTION OF THE DRAWINGS

Figure 1 is a schematic diagram of the new microspheres with lower antibody steric hindrance than conventional latex microspheres.
Figure 2 is the schematic diagram of latex microsphere-NTA-Ni preparation.
Figure 3 is the schematic diagram of the latex microsphere-NTA-Ni-Fc receptor bound to the Fc end of the anti-tacrolimus monoclonal antibody.
Figure 4 is the results of the sensitivity and linearity performance tests under different conditions for experimental groups 1 to 7.
Figure 5 is the comparison of calibration curves after coating with different N-NTA concentrations.
Figure 6 is the results of the functional sensitivity test of the kit.
Figure 7 is the Passing and Bablok regression curve of the kit of the present invention compared to LC-MS/MS.
Figure 8 is the Bland-Altman absolute deviation plot of the kit of the present invention compared to LC-MS/MS.
Figure 9 is the Bland-Altman relative deviation plot of the kit of the present invention compared to LC-MS/MS.
Figure 10 is the Passing and Bablok regression curve of the kit of the present invention compared to Abbott CMIA.
Figure 11 is the Bland-Altman absolute deviation plot of the kit of the present invention compared to Abbott CMIA.
Figure 12 is the Bland-Altman relative deviation plot of the kit of the present invention compared to Abbott CMIA.

### DETAILED DESCRIPTION OF THE PREFERRED EMBODIMENTS

The specific examples below are used to illustrate the present invention, but the scope of the present invention is not limited by the content of the examples. The experimental methods without specifying conditions in the following examples can be selected according to conventional methods and conditions, or according to the instructions of commercial kits.

### Example 1 Preparation of tacrolimus BSA complex

20 mg of purified tacrolimus (provided by Fujian Kerui) and 8 mg of succinic anhydride were dissolved in a mixture of 0.7 mL of toluene and 1 mL of CH₂Cl₂, and placed in a vial. 5 mg of dimethylaminopyridine and 5 µL of triethylamine were added sequentially. The mixed solution was stirred under argon for 24 hours at room temperature. Thin layer chromatography (TLC) analysis of the products on SiO₂ plates using CH₂Cl₂/MeOH (1: 12 v/v) as developing solvent showed that at least 95% of tacrolimus was carboxylated. The crude was then washed twice with 0.1 N HCl. The bottom CH₂Cl₂ layer was separated and washed with saturated NaCl solution. The light yellow upper organic layer was collected, dried over Mg₂SO₄, and evaporated and dried with argon stream to obtain 18.3 mg of light yellow solid for protein coupling without further purification. 4.5 mg of 1-ethyl-3-(3-dimethylaminopropyl)-carbodiimide hydrochloride (EDC) and 15 mg of N-hydroxybutanediimide (NHS) were added to 0.5 ml of anhydrous dicarboxamide containing 5 mg of carboxylated tacrolimus. After 30 min of reaction, the mixture was added dropwise to 4 ml of NaHCO₃ solution (0.1 M, pH 8.5) containing 20 mg of BSA and incubated for 120 min. The prepared tacrolimus-BSA complex was purified by gel filtration on Sepharose CL-6B column (2.5x55 cm) and monitored for elution with AKTA-prime Plus (elution buffer was 50 mM Tris-HCl, pH 8.0 containing 0.9% NaCl and 0.05% NaN₃).

### Example 2 Preparation of tacrolimus monoclonal antibody

The following antibodies were self-made from cost consideration; commercially available antibodies can achieve the following effects as long as their type corresponds to the specific Fc receptor protein, and it is not necessary to use the self-made antibodies of the present invention.

For the development of anti-tacrolimus monoclonal antibodies, tacrolimus-KLH complex (BSA replaced with KLH, keyhole hemocyanin, from Thermo, according to the method described above) were prepared for use as immunogen by a similar procedure as described above.

The monoclonal antibodies used in the examples were developed on Balb/c mice with tacrolimus-KLH complex as the immunogen. The first intradermal immunization was performed using tacrolimus- KLH complex (as immunogen) in a 100 µL emulsion prepared with Fuchs' complete adjuvant, and the booster immunization was performed 25 d later using 30 µg of immunogen in a 100 µL emulsion prepared with Fuchs' incomplete adjuvant. Two more intradermal immunizations with 30 µg of immunogen were performed at 4-week intervals, and then 30 µg of immunogen was used for intravenous and intraperitoneal pre-fusion promotion in 100 µL of Hanks' Balanced Salt Solution (China Bishi Biotechnology Co., Ltd.) (the IgM antibody strains in the examples were obtained by fusion in the first immunization). Standard fusion techniques were used to generate hybridoma cell lines secreting tacrolimus-specific antibodies. After extensive screening, several clones of anti-tacrolimus antibodies were selected with better specificity for the development of the tacrolimus determining kit.

Testing of antibody subtypes: several antibodies selected with better specificity to tacrolimus were subtyped using a commercially available antibody subtyping kit (ELISA), and the results are shown in Table 1:

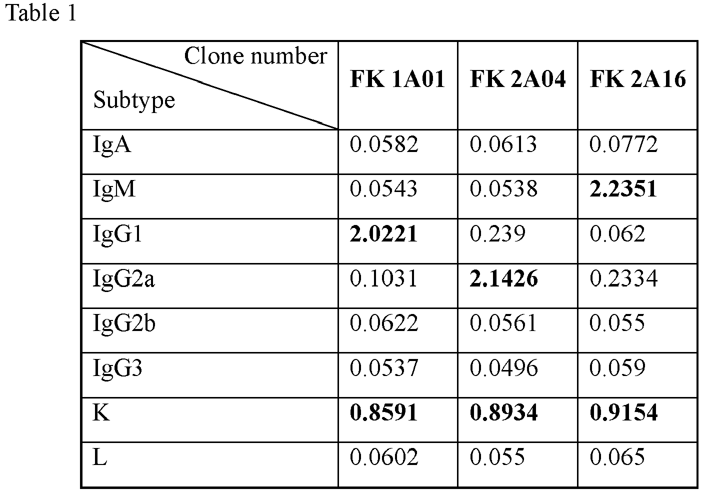

The antibody with clone number FK1A01 is of IgG1 type, the antibody with clone number FK2A04 is of IgG2a type and the antibody with clone number FK2A16 is of IGM type.

### Example 3 Screening of the antibody for coating microspheres and the pH of the microsphere storage solution.

It is found in the present invention that if Fc-yR receptor is used, it can not bind the antibody, FcAMR can be used with better results. Therefore, two different types of Fc receptor proteins (FcγR1 and FcAMR) were used to coat these three antibody types, and the preparation steps of reagent R2 (sensitizing latex reagent) in Table 2 are as follows:
(1) 70 mg of carboxyl latex microspheres (309 nm, from MBL BEIJING) was dispersed in 7 mL of MES (pH 6.1) (1% microsphere concentration), and 70 mg of NHS (N-hydroxybutanediimide) and 7 mg of EDC (1-ethyl-(3-dimethylaminopropyl) carbodiimide) were added, and the reaction was performed for 1 h at room temperature.
(2) centrifuging at 12000 rmp for 30 min and the supernatant was discarded, and the precipitation was resuspended with ultrasound in MES (pH 6.1).
(3) 1.0 mg/mL N-(5-amino-1-carboxypentyl) iminodiacetic acid was added, stirred and reacted at room temperature (1 h).
(4) 50 mmol of ethanolamine was added and the block-reaction was performed at room temperature (1 h).
(5) centrifuging at 12000 rmp for 30 min and the supernatant was discarded, and the precipitation was resuspended with ultrasound in 7 mL of 100 mM PBS (pH7.4), and the latex microsphere (PS)-NTA was prepared.
(6) excessive Ni(CH₃COO)₂ was added, stirred and reacted for 2 h.
(7) centrifuging at 12000 rmp for 30 min and the supernatant was discarded, and the precipitation was resuspended with ultrasound in 7 mL of 100 mM PBS (pH7.4), and the product was divided into two groups, #1: 3 mL, #2: 4 mL.
(8) #1 was added with 0.3 mg of FcγR1 (murine, His tagged) and #2 was added with 0.4 mg of FcAMR (murine, His tagged) in PS-NTA-Ni solution, and were shaking reacted at room temperature (2h) (as shown in Figure 3).
(9) centrifuging at 12000 rmp for 30 min and the supernatant was discarded, and the precipitation was resuspended with ultrasound in PBS, washed twice repeatedly, and dissolved in 2 mL of 100 mM PBS (pH7.4) for later use.
   The above PS-NTA-Ni-His-FcγR1 solution was equally divided into 3 groups, noting as experimental group 1, experimental group 2, and experimental group 3. The PS-NTA-Ni-His-FcAMR solution was equally divided into 4 groups, noting as experimental group 4, experimental group 5, experimental group 6, and experimental group 7.
(10) FK1A01 (IgG1 type) was added to the above experimental groups 1 and 4, FK2A04 (IgG2a type) was added to experimental groups 2 and 5, and FK2A16 (IgM type) was added to experimental groups 3, 6, and 7, with an amount of 0.2 mg of antibody in each group, and the mixture was then stirred and reacted at room temperature for 1 h.
(11) centrifuging at 12000 rmp for 30 min and the supernatant was discarded, and the precipitation was resuspended with ultrasound in PBS, washed twice repeatedly, and dissolved in 10 mL of microsphere storage solution (experimental groups 1-6 were all stored in 100 mM Tris pH7.4, 2%NaCl, 0.2% glycine, 0.1% Procline300; experimental group 7 was stored in 100 mM MES pH6.1, 2% NaCl, 0.2% glycine, 0.1% Procline300).

Reagent R1 (drug protein complex reagent) formulated in Table 2 was then prepared and saline was used as a sample for testing experiments of the concentration of protein complex in R1:

**Table 2**

| Experi mental group | Ingredient | Experim ental group 1 | Experim ental group 2 | Experim ental group 3 | Experim ental group 4 | Experim ental group 5 | Experim ental group 6 | Experim ental group 7 |
|---|---|---|---|---|---|---|---|---|
| R1 | Buffer | Tris 50mMol pH7.0 | | | | | | |
| | stabilizer | Trehalose 2% | | | | | | |
| | coagulant | PEG6000 2% | | | | | | |
| | preservativ e | Procline300 0.1% | | | | | | |
| | protein complex | **Different concentration gradients from 0 to 1600ng/mL were prepared for testing.** | | | | | | |
| R2 | Buffer | Tris 100mMo 1 pH7.4 | Tris 100mMo 1 pH7.4 | Tris 100mMo 1 pH7.4 | Tris 100mMo 1 pH7.4 | Tris 100mMo 1 pH7.4 | Tris 100mMo 1 pH7.4 | MES 100mMo 1 pH6.1 |
| | ionic stabilizer | Sodium chloride 2% | Sodium chloride 2% | Sodium chloride 2% | Sodium chloride 2% | Sodium chloride 2% | Sodium chloride 2% | Sodium chloride 2% |
| | microspher e stabilizer | Glycine 0.2% | Glycine 0.2% | Glycine 0.2% | Glycine 0.2% | Glycine 0.2% | Glycine 0.2% | Glycine 0.2% |
| | preservativ e | Procline 300 0.1% | Procline 300 0.1% | Procline 300 0.1% | Procline 300 0.1% | Procline 300 0.1% | Procline 300 0.1% | Procline 300 0.1% |
| | latex microspher e type | **PS-NTA-Ni-FcγR-antibody (Using the sensitization method of the present invention)** | | | **PS-NTA-Ni-FcAMR-Antibody (Using the sensitization method of the present invention)** | | | |
| | Monoclon al antibody used | **FK 1A01** | **FK 2A04** | **FK 2A16** | **FK 1A01** | **FK 2A04** | **FK 2A16** | **FK 2A16** |

Test was conducted on Hitachi 7180, and the specific parameter settings were as shown in Table 3:

**Table 3**

| wavelength | 546nm/none (primary/secondary) | sample | 15µL | reaction method | two-point endpoint method |
|---|---|---|---|---|---|
| optical diameter of colorimetric cup | 1cm | R1 | 100µL | calibration type | Spline/Logitlog(4p) |
| reaction temperature | 37°C | R2 | 100µL | reaction direction | up |

The device parameters were set according to the data in Table 4:

**Table 4**

| | | |
|---|---|---|
| | calibrator | determination |
| calibrator | 15µL | - |
| sample | - | 15µL |
| R1 | 100µL | 100µL |

| Mix well and incubate at 37°C for 3-5 minutes | | |
|---|---|---|
| R2 | 100µL | 100µL |
| Mix well, incubate at 37°C for about 20 seconds, read the absorbance value A1, incubate for about 5 minutes, read the absorbance value A2, calculate ΔA=A2-A 1 | | |

The test results were shown in Table 5:

**Table 5**

| protein complex Tacroli mus-BSA concen tration | Experimen tal group 1 | Experimen tal group 2 | Experimen tal group 3 | Experimen tal group 4 | Experimen tal group 5 | Experimen tal group 6 | Experimen tal group 7 |
|---|---|---|---|---|---|---|---|
| Not contai ned | 18 | 24 | 17 | 16 | 12 | 28 | 8 |
| 25ng/ mL | 91 | 122 | 59 | 57 | 23 | 186 | 21 |
| 50ng/ mL | 187 | 249 | 15 | 116 | 37 | 343 | 48 |
| 100ng/ mL | 388 | 518 | 34 | 152 | 50 | 658 | 102 |
| 200ng/ mL | 779 | 1038 | 57 | 144 | 47 | 1237 | 178 |
| 400ng/ mL | 1521 | 2027 | 45 | 115 | 43 | 2351 | 280 |
| 600ng/ mL | 2141 | 2855 | 91 | 124 | 115 | 3424 | 360 |
| 800ng/ mL | 2783 | 3710 | 22 | 115 | 21 | 4371 | 421 |
| 1000n g/mL | 3083 | 4277 | -16 | 148 | 20 | 5201 | 380 |
| 1200n g/mL | 3118 | 4724 | 9 | 82 | 15 | 6022 | 321 |
| 1400n g/mL | 3052 | 4937 | 8 | 81 | 104 | 6805 | 287 |
| 1600n g/mL | 2830 | 4947 | -18 | 56 | 83 | 7664 | 251 |

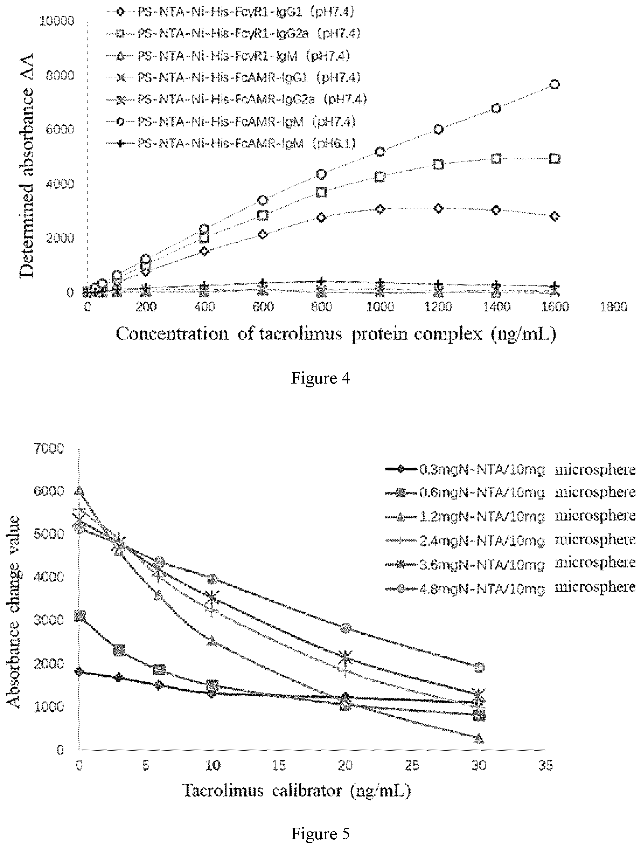

Based on the data from Table 5 and Figure 4, the results from experimental groups 1, 2, and 3 indicated that when FcγR1 was used as the receptor, it can specifically bind to FK1A01 (IgG1 type) and FK2A04 (IgG2a type) monoclonal antibodies, with a higher sensitivity and linearity observed for FK2A04 (IgG2a type). The data from experimental groups 4, 5, and 6 showed that when FcAMR was used as the receptor, it exhibits higher sensitivity and linearity in response to FK2A16 (IgM type), but does not react with FK1A01 (IgG1 type) and FK2A04 (IgG2a type).

Experimental group 6 and experimental group 7 had the same conditions except for the different pH in final storage. The data showed that the reaction decreases extremely fast under the condition of pH 6.1, and Ni is very easy to disattach under acidic condition, so the storage solution was chosen to be pH 7.4.

### Example 4 Screening experiment of N-NTA dosage in coating process

Figure 5 showed the experimental data of the optimal dosage of coating N-(5-amino-1-carboxypentyl) iminodiacetic acid (N-NTA). Other coating parameters were consistent with experimental group 6 in Example 3. The reaction absorbance value and the exhibited inhibition rate of the obtained calibration curve are optimal when the N-NTA coating concentration is 1.2 mg/10 mg microspheres.

### Example 5 Preparation of tacrolimus determining kit

Optimized reagents:
Drug protein complex reagent (Reagent R1) contained 2% trehalose, 2.5% PEG8000, 0.02% Tween20, 0.1% NaN₃ in HEPES buffer (50 mM, pH 6.8) and 1000 ng/mL tacrolimus protein complex;
Sensitized latex reagent (Reagent R2) contained 2% sodium chloride, 0.1% NaN₃ in PBS buffer (100 mM, pH 7.4) and 0.1% anti-tacrolimus antibody sensitized latex microsphere (carboxylated latex microsphere -NTA-Ni-FcAMR-FK2A16);
The calibrators contained 0.05% gentamicin, 0.1% NaN₃ in EDTA-anticoagulated bovine whole blood matrix, and purified tacrolimus of corresponding concentration the concentrations of the calibrators were divided into six points, A (0 ng/mL), B (3 ng/mL), C (6 ng/mL), D (10 ng/mL), E (20 ng/mL), and F (30 ng/mL);
The sample pre-treatment agent contained Tris-HCl buffer (50 mM, pH 7.4) with 5 mol uric acid, 3 mol/L guanidine hydrochloride, 5 U/mL subtilisin, 0.1% SDS, and 0.2% Tx100;
The drug protein complex reagent (Reagent R1) and the sensitized latex reagent (Reagent R2) prepared above were divided into vials in a volume ratio of R1:R2 = 1:1, and in combination with the calibrators and the sample pre-treatment agent to form a kit.

### Loaded for analysis:

Before the determination of samples, tacrolimus was extracted from EDTA whole blood by using the sample pre-treatment agent: 200 µL of whole blood samples or calibrators were transferred to a 1.5 mL EP tube containing 200 µL sample pre-treatment agent, vigorously vortexed for 10 seconds and bathed in water at 42°C for 10 minutes; then centrifuged at 13000 rpm for 5 minutes, and the supernatant was poured into a sample cup, and transferred to a biochemical analyzer to determine tacrolimus.

Before testing, the device parameters were set according to the data in Table 6:

**Table 6**

| wavelength | 546nm/none (primary/secondary) | sample | 15µL | reaction method | two-point endpoint method |
|---|---|---|---|---|---|
| optical diameter of colorimetric cup | 1cm | R1 | 100µL | calibration type | Spline/Logitlog(4p) |
| reaction temperature | 37°C | R2 | 100µL | reaction direction | up |

The device parameters were set according to the data in Table 7:

**Table 7**

| | | |
|---|---|---|
| | calibrator | determination |
| calibrator | 15µL | - |
| sample | - | 15µL |
| R1 | 100µL | 100µL |

| Mix well and incubate at 37°C for 3-5 minutes | | |
|---|---|---|
| R2 | 100µL | 100µL |
| Mix well, incubate at 37°C for about 20 seconds, read the absorbance value A1, incubate for about 5 minutes, read the absorbance value A2, calculate ΔA=A2-A 1 | | |

### Functional sensitivity and precision:

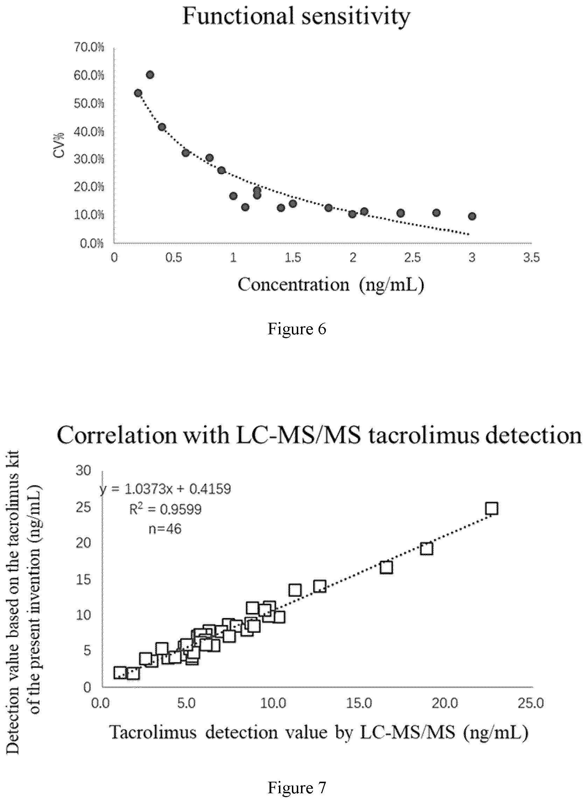

Clinical sample trays were prepared, and clinical samples of the same blood type were mixed with each other into 19 low-concentration sample trays, which were packaged after being mixed well. The samples of each concentration were repeatedly detected for 5 times, twice a day for 10 days, and the average concentration and %CV of each concentration were calculated. Based on the obtained data, the Average multiple curves fitting was applied to fit the data. The functional sensitivity of tacrolimus was defined as the tacrolimus concentration corresponding to the coefficient of variation (CV, %) of 20%. The CVs of samples were all less than 20% when the concentration more than 1.0 ng/mL, i.e., the functional sensitivity of the reagent is 1.0 ng/mL. Among them, the inter-assay precision distributions corresponding to the mixed samples at 2.1 ng/mL and 3.0 ng/mL were 11.4% and 9.5%, respectively, as shown in Figure 6.

### Dilution linearity:

In the dilution linearity study, tacrolimus calibrator A was used to dilute high-concentration tacrolimus whole blood samples. The concentration of tacrolimus in each diluted sample was then determined and the average percent recovery rate (%) was calculated. The research data were shown in Table 8 below:

**Table 8**

| dilution coefficient | detection concentration (ng/mL) | | | | calculated concentration (ng/mL) | recovery rate (%) |
|---|---|---|---|---|---|---|
| | n1 | n2 | n3 | average value | | |
| undiluted | 29.49 | 29.04 | 28.56 | 29.03 | - | - |
| 1:1.11 | 25.03 | 26.90 | 26.23 | 26.05 | 28.92 | 100 |
| 1:1.25 | 23.18 | 22.70 | 23.41 | 23.10 | 28.87 | 99 |
| 1:1.43 | 21.12 | 20.53 | 20.46 | 20.70 | 29.61 | 102 |
| 1:1.67 | 18.05 | 17.59 | 17.52 | 17.72 | 29.59 | 102 |
| 1:2.5 | 11.29 | 11.41 | 11.59 | 11.43 | 28.57 | 98 |
| 1:5 | 5.79 | 5.62 | 5.79 | 5.73 | 28.67 | 99 |
| 1:10 | 3.10 | 3.18 | 2.78 | 3.02 | 30.20 | 104 |

| | | | | | | |
|---|---|---|---|---|---|---|
| Average recovery rate: 101% Calculated value = Detected concentration × Diluted concentration Recovery rate % = (calculated concentration/pre-dilution concentration) × 100 | | | | | | |

### Example 6 Comparison of the determination of tacrolimus in clinical sampleswith the tacrolimus kit of the present invention, Abbott CMIA and LC-MS/MS

Forty-six whole blood EDTA samples were used for comparative study, and the concentrations of tacrolimus in these samples were determined by tacrolimus kit based on the present invention, Abbott CMIA and LC-MS/MS respectively. Among them, ABBOTT Tacrolimus CMIA was carried out in strict accordance with the kit instructions on the Architect I1000 analyzer.

### LC-MS/MS was performed as described below:

LC-MS/MS: LC-MS/MS analysis was performed using a SCIEX Triple QuadTM 4500MD-Jasper system (AB SCIEX). 200 µL of each sample and calibrator were transferred into a 2 mL EP tube, followed by the addition of 1000 µL of acetonitrile containing 5 ng of diazepam (internal standard). The mixture was vortexed for 1 minute and then centrifuged at 13000×g for 5 minutes. 600 µL of the supernatant were transferred to a 2 mL EP tube, mixed with 600 µL of pure water, vortexed for 10 seconds, and then centrifuged again at 13000×g for 5 minutes. The supernatant was transferred to an autosampler vial, and 10 µL of the supernatant sample was injected into the LC-MS/MS system. Tacrolimus was separated on a Hypersil Gold C-18 column (3 µm, 2.1 × 100 mm) with a gradient mobile phase consisting of solvent A (acetonitrile) and solvent B (pure water). The flow rate was 0.4 mL/min for all programs. The column temperature was 45°C. The mobile phase was a mixture of 10% A and 90% B for the initial 2 minutes, then solvent A was increased to 90% and held for 3 minutes. Finally, solvent A was reduced to 10% at 5.1 min and held for 2 min; the next sample was then injected. Mass spectrometer settings: spray voltage 5.5KV, curtain gas 30psi, collision gas (CAD): 9, source GS1: 45 psi, source GS2: 45 psi, and drop temperature 400°C. The MRM m/z ratios monitored by tacrolimus and internal standard diazepam were m/z 821.5→768.5/786.6 and 285.0→7222.1, respectively. Integration and quantification of ion chromatographic peaks was performed using a TRIPLE QUADTM 4500MD system.

The sample comparison data were shown in Table 9:

**Table 9**

| Sample number | LC-MS tacrolimus | Abbott CMIA tacrolimus | Tacrolimus kit based on the present invention |
|---|---|---|---|
| 1 | 5.5 | 6.1 | 7.1 |
| 2 | 5.5 | 6 | 6.9 |
| 3 | 6.2 | 6.7 | 7.8 |
| 4 | 10.2 | 10.6 | 9.8 |
| 5 | 2.9 | 2.9 | 3.6 |
| 6 | 6.0 | 6.4 | 7.3 |
| 7 | 7.0 | 7.2 | 7.7 |
| 8 | 4.7 | 5.2 | 5.6 |
| 9 | 4.7 | 4.8 | 4.6 |
| 10 | 7.3 | 7.7 | 8.7 |
| 11 | 3.8 | 3.8 | 4.1 |
| 12 | 9.7 | 10 | 11.2 |
| 13 | 7.7 | 7.8 | 8.5 |
| 14 | 12.6 | 13.5 | 14.1 |
| 15 | 2.5 | 2.7 | 4 |
| 16 | 5.7 | 6.2 | 7.3 |
| 17 | 1.0 | 1.1 | 2 |
| 18 | 5.0 | 5 | 5.9 |
| 19 | 6.9 | 7.5 | 7.7 |
| 20 | 5.7 | 6 | 7.3 |
| 21 | 18.8 | 19 | 19.2 |
| 22 | 22.6 | 25.6 | 24.8 |
| 23 | 8.4 | 8.8 | 8 |
| 24 | 16.5 | 18.9 | 16.7 |
| 25 | 5.2 | 5.2 | 4 |
| 26 | 4.7 | 5.1 | 4.5 |
| 27 | 6.5 | 6.6 | 5.8 |
| 28 | 5.2 | 5.2 | 4.3 |
| 29 | 9.6 | 10.2 | 9.9 |
| 30 | 6.0 | 6.3 | 6.6 |
| 31 | 6.9 | 7.4 | 7.7 |
| 32 | 7.4 | 7.5 | 7.1 |
| 33 | 8.7 | 11 | 11.1 |
| 34 | 5.9 | 6 | 6.1 |
| 35 | 5.0 | 5.2 | 5.4 |
| 36 | 8.6 | 8.5 | 8.9 |
| 37 | 11.2 | 13.2 | 13.5 |
| 38 | 5.7 | 5.9 | 6.2 |
| 39 | 8.8 | 8.8 | 8.5 |
| 40 | 3.4 | 5.7 | 5.4 |
| 41 | 4.2 | 4.2 | 4.2 |
| 42 | 1.8 | 1.9 | 1.9 |
| 43 | 6.0 | 6 | 5.9 |
| 44 | 5.3 | 5.4 | 4.8 |
| 45 | 9.4 | 10.4 | 10.8 |
| 46 | 4.9 | 6 | 5.9 |

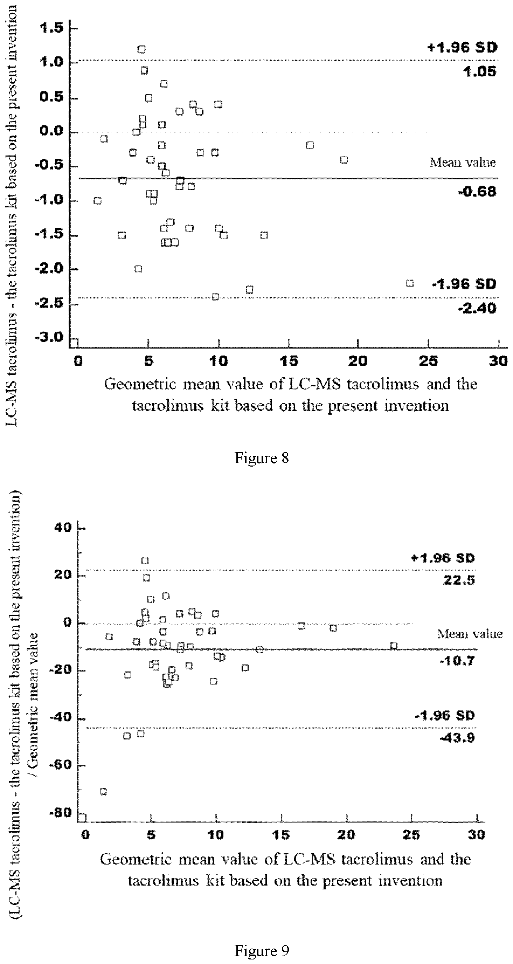

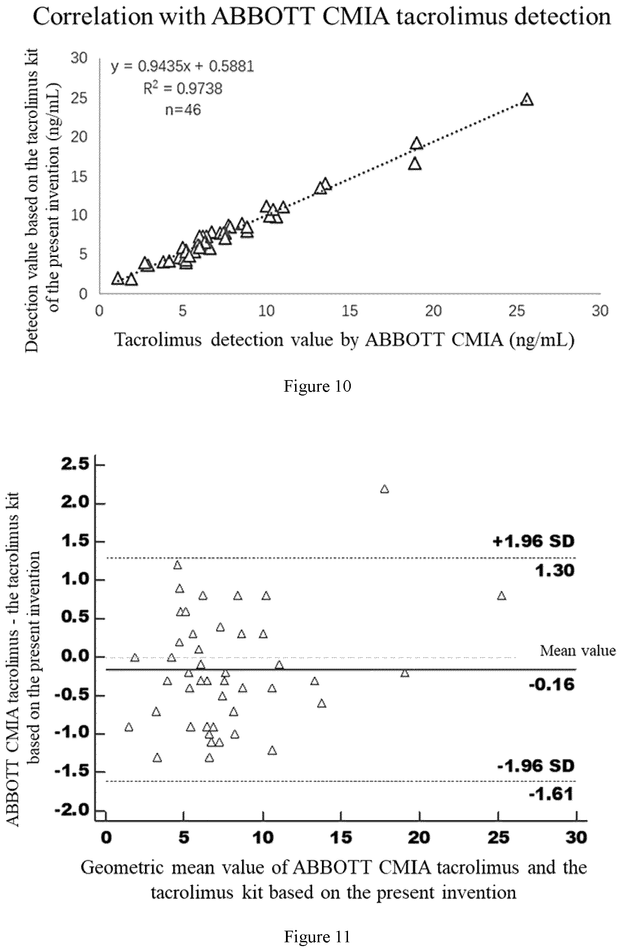

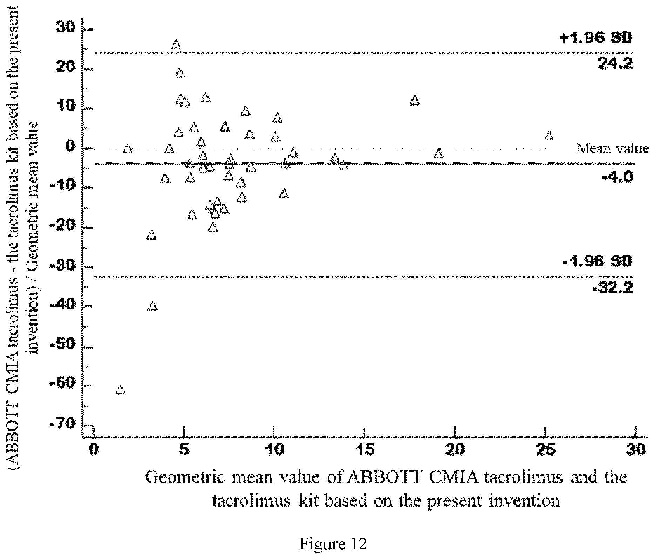

Linear regression and Bland-Altman analysis were performed on the tacrolimus concentrations in samples tested with the tacrolimus kit based on the present invention, LCMS/MS, and ABBOTT CMIA. The linear regression equations were Y = 1.0373X + 0.4159 (R²= 0.9599) and Y= 0.9435X + 0.5881 (R² = 0.9738), as shown in Figure 7 and Figure 10, respectively. The average absolute deviations of LC-MS/MS and ABBOTT CMIA to the tacrolimus kit of the present invention were -0.68 ng/mL and -0.16 ng/mL, respectively, with relative deviations of -10.7% and -4.0%, as shown in Figures 8 and 9, and Figures 11 and 12, respectively.

As can be seen from the above examples, the tacrolimus determining kit prepared by the study of the present invention has high sensitivity and can be industrially produced in large-scale. Compared to methods such as HPLC-MS, it offers superior convenience in detection and meets domestic needs.

The aforementioned description of the examples is to facilitate the understanding and use of the invention by a person of ordinary skill in the art. It is evident to those skilled in the art that various modifications can be made to these examples without undue labor, and the general principles set forth herein can be applied to other examples without the need for creative work. Accordingly, the present invention is not limited to the examples described above. Improvements and modifications made by those skilled in the art based on the disclosure of the present invention, without departing from the scope of the invention, should be within the protection of the present invention.

## Claims

1. A kit for quantitatively determining tacrolimus, comprising a sensitizing latex reagent, wherein the sensitizing latex reagent comprising a latex microsphere-NTA-Ni-Fc receptor-anti-tacrolimus monoclonal antibody.

2. The kit of claim 1, wherein the preparation method of the latex microsphere-NTA-Ni-Fc receptor-anti-tacrolimus monoclonal antibody comprises following steps:
i) binding a latex microsphere covalently with N-(5-amino-1-carboxypentyl) iminodiacetic acid to produce latex microsphere-NTA;
ii) binding the latex microsphere-NTA with a Ni ion to produce latex microsphere-NTA-Ni;
iii) binding the latex microsphere-NTA-Ni with a His-tagged Fc receptor to produce latex microsphere-NTA-Ni-Fc receptor;
iv) binding the latex microsphere-NTA-Ni-Fc receptor with the Fc end of an anti-tacrolimus monoclonal antibody to produce the latex microsphere-NTA-Ni-Fc receptor-anti-tacrolimus monoclonal antibody.

3. The kit of claim 2, wherein the preparation of the latex microsphere-NTA of i) comprises following steps:
(1) dispersing the latex microsphere with a particle size of 50 nm to 350 nm in MES with a pH of 5.8 to 6.5, making the microsphere mass volume percentage at 1%, then adding N-hydroxysuccinimide and 1-ethyl-(3-dimethylaminopropyl) carbodiimide at a coating concentration of 0.1 to 3 mg/10 mg of microsphere, and reacting at room temperature for 0.5 to 3 hours;
(2) centrifuging anddiscarding the supernatant, and resuspending in MES with pH of 5.8 to 6.5;
(3) adding 0.5 to 3 mg/mL of N-(5-amino-1-carboxypentyl)iminodiacetic acid and reacting for 1-3h;
(4) adding 30 to 50 mmol primary amine and block-reacting for 1-3 h;
(5) centrifuging and discarding the supernatant, and resuspending in PBS at a pH of 6.8 to 8.5, to produce the latex microsphere-NTA;
preferably, the coating concentration of N-hydroxysuccinimide and 1-ethyl-(3-dimethylaminopropyl)carbodiimide is 1.2 mg/10 mg of microsphere.

4. The kit of claim 2, wherein the preparation of the latex microsphere-NTA-Ni of ii) comprises following steps:
(1) adding Ni(CH₃COO)₂ and reacting for 0.5-3 h;
(2) centrifuging and discardingthe supernatant, and resuspending in 0.1 Mol PBS at a pH of 6.8 to 8.5.

5. The kit of claim 2, wherein the preparation of the latex microsphere-NTA-Ni-Fc receptor of iii) comprises following steps:
(1) adding the His-tagged Fc receptor to the latex microsphere-NTA-Ni and reacting for 0.5 to 3 hours;
(2) centrifuging and discarding the supernatant, suspending and washing with PBS and dissolving in PBS;
preferably, the His-tagged Fc receptor is a 6×His-Fc receptor.

6. The kit of claim 2, wherein the preparation method of the latex microsphere-NTA-Ni-Fc receptor-anti-tacrolimus monoclonal antibody of iv) comprises following steps:
(1) adding the anti-tacrolimus monoclonal antibody to the latex microsphere-NTA-Ni-Fc receptor, making the antibody coating amount of the latex microsphere at 0.05 to 10 mg of anti-tacrolimus monoclonal antibody /10 mg of latex microsphere, and reacting for 1 hour;
(2) centrifuging and discarding the supernatant, suspending and washing with PBS, and dissolving in a microsphere storage solution;
preferably, the antibody coating amount of the latex microsphere is 0.1 to 0.4 mg of anti-tacrolimus monoclonal antibody /10 mg of latex microsphere.

7. The kit of claim 6, wherein the microsphere storage solution comprises a buffer with a concentration of 50-500 mM and a pH of 6.5-7.8, an ionic stabilizer with a mass percentage of 0.5-5%, a microsphere stabilizer with a mass percentage of 0.05-0.5%, and a preservative;
the mass percentage of the ionic stabilizer is preferably 2%;
the mass percentage of the microsphere stabilizer is preferably 0.1%- 0.2%.

8. The kit of claim 7, wherein the ionic stabilizer is NaCl;
and/or, the microsphere stabilizer is glycine.

9. The kit of claim 1, wherein the kit further comprises a drug-protein complex reagent, which comprises a tacrolimus drug-protein complex, a stabilizer, a coagulant promoter, a surfactant, a preservative and a buffer;
the concentration of the tacrolimus drug-protein complex is preferably 0.1 µg/mL-3 µg/mL;
the concentration of the buffer is preferably 50-500 mM, and the pH is 6.5-7.8;
the mass percentage of the stabilizer is preferably 1%- 10%;
the mass percentage of the surfactant is preferably 0.01%- 0.5%.

10. The kit of claim 1, wherein the kit further comprises a calibrator, which comprises a preservative, a tacrolimus pure product, and a bovine whole blood matrix, and the concentration of tacrolimus in the calibrator is selected from 5 to 7 points between 0-50 ng/mL; preferably, the concentrations of tacrolimus in the calibrator are 0 ng/mL, 3 ng/mL, 6 ng/mL, 10 ng/mL, 20 ng/mL, and 30 ng/mL, respectively, 6 points in total.

11. The kit of claim 1, wherein the kit further comprises a sample pre-treatment agent, which comprises a protein denaturant, a protein hydrolase, a surfactant and a buffer;
the buffer is Tris-HCl buffer with a pH of 6.5-8.5;
the protein denaturant is uric acid of 3 mol/L-8 mol/L, and/or guanidine hydrochloride of 2 mol/L-6 mol/L;
the protein hydrolase is subtilisin of 2.5 U/ml-10 U/ml.

12. A method for quantitatively determining the concentration of tacrolimus in a sample, wherein the method comprises using the kit of any one of claims 1-11, and the method comprises following steps:
(1) mixing and incubating the sample or the calibrator with the sample pre-treatment agent, and centrifuging to obtain a supernatant;
(2) mixing the supernatant in (1) with the drug-protein complex reagent and incubating to obtain a mixture;
(3) adding the sensitizing latex reagent to the mixture in (2), reading the absorbance as A1, incubating and reading the absorbance as A2;
(4) calculating the absorbance increment ΔA of the sample or the calibrator, ΔA = A2-A1;
(5) establishing a calibration curve with the absorbance increment ΔA of the calibrator against the concentration of tacrolimus contained, and achieving quantitative detection of the concentration of tacrolimus in the sample by comparing the absorbance increment ΔA of the sample.
